(19) Europäisches Patentamt
European Patent Office
Office européen des brevets

(11) Veröffentlichungsnummer: **0 026 848**
**B1**

(12) # EUROPÄISCHE PATENTSCHRIFT

(45) Veröffentlichungstag der Patentschrift: **04.05.83**

(21) Anmeldenummer: **80105275.4**

(22) Anmeldetag: **04.09.80**

(51) Int. Cl.³: **C 07 C 91/30,** C 07 C 93/26, C 07 C 101/18, C 07 C 103/50, C 07 C 117/00, C 07 C 121/43, C 07 C 125/065, C 07 C 127/15, C 07 C 143/833, C 07 C 147/02, C 07 C 149/24

(54) **Tetralinderivate, ihre Herstellung und Heilmittel, welche diese Verbindungen enthalten.**

(30) Priorität: **14.09.79 CH 8347/79**
**18.07.80 CH 5547/80**

(43) Veröffentlichungstag der Anmeldung: **15.04.81 Patentblatt 81/15**

(45) Bekanntmachung des Hinweises auf die Patenterteilung: **04.05.83 Patentblatt 83/18**

(84) Benannte Vertragsstaaten: **AT BE CH DE FR GB IT LI LU NL SE**

(56) Entgegenhaltungen:
**DE-A-2 333 847**
**DE-A-2 623 417**
**DE-A-2 803 582**
**US-A-3 991 207**

(73) Patentinhaber: **SANDOZ AG, Lichtstrasse 35, CH-4002 Basel (CH)**

(72) Erfinder: **Seiler, Max Peter, Dr., Clarastrasse 21, CH-4058 Basel (CH)**
Erfinder: **Stoll, André, Dr., Rheinparkstrasse 3, CH-4127 Birsfelden (CH)**

**EP 0 026 848 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Tetralinderivate, ihre Herstellung und Heilmittel, welche diese Verbindungen enthalten

Die Erfindung betrifft neue Tetralinderivate, ihre Herstellung und pharmazeutische Präparate, welche diese Verbindungen enthalten.

Die deutsche Offenlegungsschrift Nr. 2 333 847 betrifft eine sehr breite Klasse von Tetrahydronaphtholen, welche als Weichmacher von Wasser, als Korrosionsinhibitoren in Schmierstoffen, als ZNS-dämpfende Mittel, als Mittel zur Behandlung von Herzmuskelflimmern und von Herzarrhythmie bei Warmblütern, sowie zur Senkung des Blutdrucks und als Desinfektionsmittel verwendet werden können. Diese Tetrahydronaphthole können eine große Anzahl Substituenten sowohl in den aromatischen als in den hydrierten Kern tragen. Die Hydroxygruppe am aromatischen Kern kann frei oder verestert sein, der hydrierte Sechsring kann u. a. eine nicht cyclische disubstituierte Aminogruppe tragen. Als Substituenten am N-Atom dieser Aminogruppe werden erwähnt: Alkyl, Alkenyl, Hydroxyalkyl und Phenylalkyl, jedoch als einzige asymmetrisch disubstituierte Aminogruppe nur die N-methyl-N-Benzylaminogruppe genannt und durch Beispiele belegt.

Es wurde nun gefunden, daß bestimmte Tetrahydronaphthole, welche in der o. e. DOS nicht spezifisch vorgeschlagen oder beschrieben werden, wertvolle pharmakologische Eigenschaften besitzen.

Die vorliegende Erfindung bezieht sich nämlich auf neue 2-Amino-5-hydroxy-1,2,3,4-tetrahydronaphthaline, welche am N-Atom der Aminogruppe zwei Alkylgruppen enthalten, wovon eine unsubstituiert ist und die andere mindestens eine funktionelle Gruppe trägt, sowie deren physiologisch hydrolysierbare Ester und die Säureadditionssalze dieser Verbindungen.

Diese Verbindungen werden weiter im Text als die erfindungsgemäßen Verbindungen bezeichnet.

Unter funktionelle Gruppe ist hier jede in der organischen Chemie übliche reaktive Gruppe zu verstehen, inklusive Halogenatome und ungesättigte C—C-Gruppen, jedoch exklusive aromatische Ringe. Hierbei ist noch zu bemerken, daß die funktionelle Gruppe direkt und nicht über einen aromatischen Ring an die Alkylgruppe gebunden sein soll.

Die Erfindung betrifft die Verbindungen der Formel I

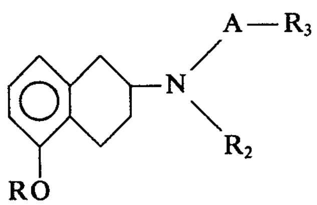

(I)

worin

| | |
|---|---|
| R | für Wasserstoff oder eine $R_1CO$-Gruppe |
| $R_1CO$ | für den unter physiologischen Bedingungen durch Hydrolyse abspaltbaren Acrylrest einer Säure |
| $R_2$ | für Alkyl mit 1 bis 4 C-Atomen |
| A | für Alkylen mit 1 bis 5 C-Atomen |
| $R_3$ | für Halogen, eine freie oder veresterte OH-Gruppe, SH, $N_3$, CN, $COR_4$, $NH-X-R_5$, Y-Alkyl mit 1 bis 4 C-Atomen, Y-Phenylalkyl mit 7 bis 10 C-Atomen, Y-Phenyl oder Alkenyl oder Alkinyl mit 2 bis 4 C-Atomen, |
| $R_4$ | für Alkoxy mit 1 bis 4 C-Atomen oder 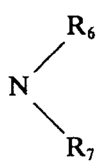 |
| $R_5$ | für Alkyl mit 1 bis 3 C-Atomen, gegebenenfalls durch Alkyl mit 1 bis 4 C-Atomen, Alkoxy mit 1 bis 4 C-Atomen, Fluor, Chlor oder Brom mono- oder disubstituiertes Phenyl, $NR_6R_7$ oder, falls X CO bedeutet, zusätzlich auch für Alkoxy mit 1 bis 4 C-Atomen |
| $R_6$ und $R_7$ | unabhängig voneinander für Wasserstoff oder Alkyl mit 1 bis 4 C-Atomen |
| X | für CO oder $SO_2$ und |
| Y | für O, S, SO oder $SO_2$ |

stehen sowie ihre Säureadditionssalze.

Die erfindungsgemäßen Verbindungen können in Form von optischen Isomeren oder in Form von Racematen oder in besonderen Fällen, z. B. falls in Formel I Y für SO steht, auch in Form von

Diastereoisomeren auftreten. Bevorzugt haben die Verbindungen die gleiche sterische Konfiguration wie die Verbindung des Beispiels 23.

A kann sowohl für eine unverzweigte als für eine verzweigte Alkylenkette stehen. Vorzugsweise steht A für eine $(CH_2)_p$-Kette, worin p 1 bis 5 bevorzugt 2 bis 4 bedeutet. Bevorzugt steht A für die $(CH_2)_3$-Gruppe

$R_1$ steht bevorzugt für Wasserstoff, Alkyl mit 1 bis 19 C-Atomen, Phenylalkyl mit 7 bis 10 C-Atomen oder gegebenenfalls durch Alkyl mit 1 bis 4 C-Atomen, Alkoxy mit 1 bis 4 C-Atomen, Fluor, Chlor oder Brom mono- oder disubstituiertes Phenyl

$R_2$ steht bevorzugt für die n-Propylgruppe

$R_3$ bedeutet vorzugsweise CN, S-Alkyl mit 1 bis 4 C-Atomen,

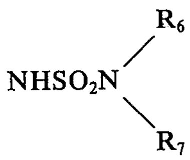

OH oder F

Falls $R_3$ eine veresterte OH-Gruppe darstellt, kann der Säurerest dieses Esters z. B. von einer Carbonsäure $R_1COOH$ oder einer Carbaminsäure, welche am N-Atom durch Kohlenwasserstoffreste mit 1 bis 10 C-Atomen substituiert sein kann, abgeleitet sein.

Die Erfindung betrifft u. a. eine Gruppe Verbindungen der Formel Ia

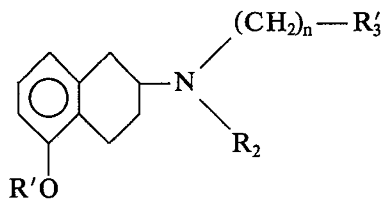

(Ia)

worin

$R'$ für Wasserstoff oder eine $R_1'CO$-Gruppe

$R_2$ für Alkyl mit 1 bis 4 C-Atomen,

$R_3$ für eine CN-, $COR_4$-,

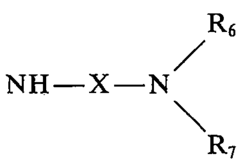

oder $NHCOR_5'$-Gruppe,

$R_1'$ für Wasserstoff, Alkyl mit 1 bis 19 C-Atomen, Phenylalkyl mit 7 bis 10 C-Atomen oder gegebenenfalls durch Alkyl mit 1 bis 4 C-Atomen, Alkoxy mit 1 bis 4 C-Atomen, Fluor, Chlor oder Brom mono- oder disubstituiertes Phenyl,

$R_4$ für Alkoxy mit 1 bis 4 C-Atomen oder

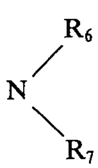

$R_6$ und $R_7$ unabhängig voneinander für Wasserstoff oder Alkyl mit 1 bis 4 C-Atomen,

$R_5'$ für Alkyl mit 1 bis 3 C-Atomen,

X für CO oder $SO_2$

n für eine ganze Zahl von 2 bis 5

stehen, sowie ihre Säureadditionssalze.

Die Erfindung betrifft auch eine Gruppe Verbindungen der Formel Ib

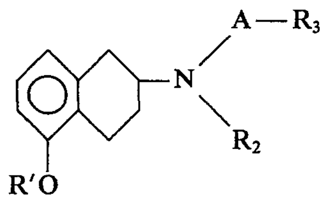

(Ib)

worin

$R'$ und $R_2$ die für die Formel Ia angegebenen Bedeutungen haben
A für Alkylen mit 1 bis 5 C-Atomen
$R_3$ für Halogen, eine freie oder veresterte OH-Gruppe, SH, $N_3$, CN, $COR_4$, $NH-X-R_5$, Y-Alkyl mit 1 bis 4 C-Atomen, Y-Phenylalkyl mit 7 bis 10 C-Atomen, Y-Phenyl, Alkenyl oder Alkinyl mit 2 bis 4 C-Atomen,
$R_4$ für Alkoxy mit 1 bis 4 C-Atomen oder

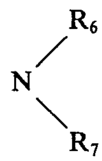

$R_5$ für Alkyl mit 1 bis 3 C-Atomen, gegebenenfalls durch Alkyl mit 1 bis 4 C-Atomen, Alkoxy mit 1 bis 4 C-Atomen, Fluor, Chlor oder Brom mono- oder disubstituiertes Phenyl, $NR_6R_7$ oder, falls X CO bedeutet, zusätzlich auch für Alkoxy mit 1 bis 4 C-Atomen
$R_6$ und $R_7$ unabhängig voneinander für Wasserstoff oder Alkyl mit 1 bis 4 C-Atomen
X für CO oder $SO_2$ und
Y für O, S, SO oder $SO_2$

stehen sowie ihre Säureadditionssalze, mit der Maßgabe, daß, falls A für $(CH_2)_n$, worin n 2 bis 5 bedeutet, steht, $R_3$ nicht CN, $COR_4$,

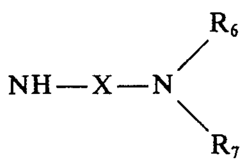

oder $NHCOR_5'$, worin $R_5'$ Alkyl mit 1 bis 3 C-Atomen

bedeutet, stehen kann sowie ihre Säureadditionssalze.

Die erfindungsgemäßen 2-Dialkylamino-1,2,3,4-tetrahydronaphthaline, mit freier OH-Gruppe in Stellung 5, oder ihre Säureadditionssalze, können z. B. hergestellt werden, indem man a) in 2-Monoalkylamino-5-hydroxy-1,2,3,4-tetrahydronaphthaline einen die funktionelle Gruppe enthaltenden Alkylrest eingeführt oder b) aus 2-Dialkylamino-5-hydroxy-1,2,3,4-tetrahydronaphthalinen, welche auf einem der am N-Atom gebundenen Alkylreste mindestens eine funktionelle Gruppe tragen und worin die 5-OH-Gruppe durch eine Schutzgruppe geschützt ist, diese Schutzgruppe entfernt. Die in Stellung 5 veresterten erfindungsgemäßen Verbindungen können durch Umsetzung der in Stellung 5 nicht veresterten Tetralinderivate mit einer unter physiologischen Bedingungen durch Hydrolyse abspaltbaren Säure oder einem reaktiven Derivat einer solchen Säure hergestellt werden (Verfahren c).

Spezifisch gelangt man

a) zu einer Verbindung der Formel I, worin der Rest R für Wasserstoff steht, und ihren Säureadditionssalzen, indem man in eine Verbindung der Formel II

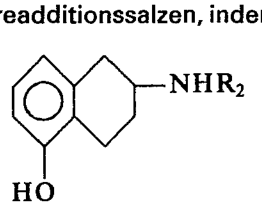

(II)

den Rest $R_3-A$ einführt, z. B. mit einer Verbindung der Formel III

$R_3-A-Z$ III

worin Z für einen unter den Reaktionsbedingungen als HZ abspaltbaren Rest steht,

b) zu einer Verbindung der Formel I, worin der Rest R für Wasserstoff steht, und ihren Säureadditionssalzen, indem man in einer Verbindung der Formel IV

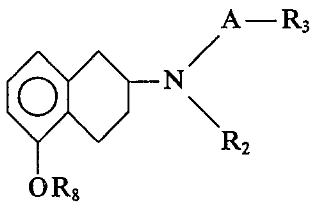

(IV)

worin $R_8$ eine Hydroxyschutzgruppe bedeutet, den Rest $R_8$ abspaltet

c) zu einer Verbindung der Formel I, worin der Rest R für die $R_1CO$-Gruppe steht, und ihren Säureadditionssalzen, indem an eine Verbindung der Formel I, worin R für Wasserstoff steht mit $R_1COOH$ oder einem reaktionsfähigen Derivat dieser Carbonsäure acyliert

und die erhaltene Verbindung der Formel I gegebenenfalls in ihre Säureadditionssalze überführt.

Die als freie Basen vorliegenden erfindungsgemäßen Verbindungen können auf an sich bekannte Weise in ihre Säureadditionssalze überführt werden und umgekehrt. So können die erfindungsgemäßen Verbindungen z. B. mit anorganischen Säuren wie Chlorwasserstoffsäure oder mit organischen Säuren wie Malonsäure, Fumarsäure, Embonsäure, Naphthalin-1,5-disulfonsäure, Maleinsäure etc. Säureadditionssalze bilden.

Das Verfahren a) kann nach an sich für die Herstellung von tertiären Aminen bekannten Methoden durchgeführt werden. Vorzugsweise werden die Verbindungen der Formeln II und III in einem inerten Lösungsmittel, wie Dimethylformanid, erhitzt. Die Kondensation kann in Gegenwart einer Base, z. B. tert. Amin oder Alkalimetallcarbonat oder -hydrogencarbonat erfolgen. Der Rest Z steht vorzugsweise für Chlor, Brom, Jod, eine Alkylsulfonyloxy- oder Arylsulfonyloxygruppe.

Das Verfahren b) kann nach an sich für die Abspaltung einer Hydroxyschutzgruppe bekannten Methoden durchgeführt werden.

$R_8$ bedeutet vorzugsweise eine niedere Alkylgruppe, insbesondere die Methylgruppe, eine Aralkylgruppe, insbesondere die Benzylgruppe oder eine Acylgruppe, insbesondere die Acetylgruppe.

Zur Abspaltung einer Alkylgruppe verwendet man vorzugsweise eine Lewissäure in einem inerten organischen Lösungsmittel, oder Alkalimetallalkylmercaptid in einem inerten polaren organischen Lösungsmittel. Zur Abspaltung einer Acylgruppe wird die Verbindung der Formel II vorzugsweise mit einer alkoholischen Halogenwasserstoffsäure erwärmt.

Das Verfahren c) kann in einer für die Acylierung von Phenolen bekannten Weise durchgeführt werden. Als reaktive Derivate der Carbonsäuren können beispielsweise Säurehalogenide oder Säureanhydride verwendet werden. Die Reaktion wird vorzugsweise in einem sauren Lösungsmittel, wie Trifluoressigsäure, oder in Gegenwart einer Base, z. B. Pyridin, durchgeführt.

Für die Herstellung von bestimmten Verbindungen, z. B. solche der Formel I, worin einer der Reste RO und $R_3$ für eine freie und der andere Rest für eine veresterte OH-Gruppe steht, können eventuell selektive Reaktionsbedingungen gewählt oder vorübergehend Schutzgruppen eingeführt werden.

Alle erwähnten Reaktionen werden zweckmäßigerweise bei Temperaturen zwischen ca. 20° bis 200° C durchgeführt.

Die erfindungsgemäßen Verbindungen können in an sich bekannter Weise isoliert und gereinigt werden.

Die optisch aktiven erfindungsgemäßen Verbindungen können z. B. ausgehend von optisch aktiven Ausgangsprodukten (hergestellt nach an sich für die Spaltung von Racematen üblichen Methoden) erhalten werden (z. B. analog zur Herstellung der Verbindung des Beispiels 23). Die Antipoden- bzw. Diastereomeren-Trennung kann auch mit den erfindungsgemäßen Verbindungen durchgeführt werden.

Die Ausgangsverbindungen z. B. die Verbindungen der Formeln II und IV sind entweder bekannt oder gehören in an sich bekannter Weise oder analog zu den in den Beispielen beschriebenen Methoden hergestellt werden.

Die Verbindungen der Formel IV können z. B. analog zum Verfahren a) hergestellt werden oder durch Umwandlung eines Restes $R_3$ in einer Verbindung der Formel IV in einen anderen Rest $R_3$, z. B. Ersatz eines Halogenatoms durch eine S-Alkylgruppe mit einem Alkylthiol oder Oxidation des S-Alkylrestes mittels Wasserstoffperoxid bzw. Natriumperjodat zur $SO_2$-Alkyl- bzw. SO-Alkyl-gruppe.

Die erfindungsgemäßen Verbindungen weisen interessante pharmakologische Eigenschaften auf und können daher als Pharmazeutika z. B. für therapeutische Zwecke verwendet werden. Insbesondere stimulieren sie die Dopaminrezeptoren.

So zeigen sie am narkotisierten Hund eine Blutdrucksenkung und Steigerung der Durchblutung in

der Arteria mesenterica, charakterisch für periphere Dopaminrezeptorenstimulation, in Dosen ab 0,1 bis 10 mg/kg i. v. Die erfindungsgemäßen Verbindungen können daher zur Behandlung von Herz-Kreislauferkrankungen, insbesondere zur Behandlung des erhöhten Blutdrucks, und zur Behandlung von Nierenversagen und bei Herzinsuffisienz verwendet werden.

Weiter zeigen diese Verbindungen eine zentrale Dopamin-rezeptoren-stimulierende Wirkung. Diese Wirkung kann an Ratten, bei denen durch eine 6-Hydroxydopamin-Injektion in die substantia nigra eine unilateriale Verletzung der nigro-neostriatalen Dopaminbahn erzeugt wurde, mit Dosen zwischen etwa 0,3 bis 5 mg/kg i.p. festgestellt werden [Methode nach U. Ungerstedt, Acta physiol. scand. Suppl. 367, 69—93 (1973)]. Nach Verabreichung des Wirkstoffes war eine deutliche Aktivierung dadurch erkennbar, daß die Ratten in Richtung der nicht denervierten Seite rotierten. Die zentralen dopaminergen Eigenschaften der neuen Verbindung wurden im Apomorphin-stereotypie-Test an der Ratte (siehe belgisches Patent Nr. 831 488) nach Verabreichung von 30 mg/kg i.p. bestätigt.

Die neuen Substanzen können aufgrund ihrer dopaminergen Eigenschaften zur Behandlung von Parkinsonismus Anwendung finden.

Ferner besitzen die erfindungsgemäßen Verbindungen eine Prolaktinsekretions-hemmende Wirkung. Diese Wirkung konnte an der männlichen Ratte in Dosen ab 0,1 bis 10 mg/kg s.c. durch Analyse der Serumprolaktinkonzentration mittels RIA-Methodik nachgewiesen werden. Die Blutentnahme erfolgte durch Dekapitation. Die Verbindungen können daher auch als Prolaktionsekretionshemmer verwendet werden.

Für alle oben genannten Anwendungen ist eine täglich zu verabreichende Menge zwischen 2 und 1000 mg angezeigt. Diese Dosis kann auch in kleineren Dosen 2—4mal täglich oder in Retardform verabreicht werden. Eine Einheitsdosis kann zwischen 0,5 und 500 mg des Wirkstoffs zusammen mit geeigneten pharmazeutisch indifferenten Hilfsstoffen enthalten.

Die Verbindungen können auch in Form ihrer pharmazeutisch geeigneten Säureadditionssalze eingesetzt werden. Diese zeigen eine ähnliche Aktivität wie die freien Basen.

Die Erfindung betrifft auch pharmazeutische Präparate, die eine erfindungsgemäße Verbindung als freie Base oder als pharmazeutisch geeignetes Säureadditionssalz enthalten. Diese pharmazeutischen Präparate, beispielsweise eine Lösung oder eine Tablette, können nach bekannten Methoden, unter Verwendung der üblichen Hilfs- und Trägerstoffe, hergestellt werden.

In den nachfolgenden Beispielen erfolgen alle Temperaturangaben in Celsiusgraden und sind unkorrigiert. Bei den angegebenen NMR-Spektren steht S für Singlet, M für Multiplet und T für Triplet. Propyl steht immer für n-Propyl.

Das Beispiel 23 betrifft die bevorzugte Verbindung.

## Beispiel 1

### 2-[N-(4-Cyanobutyl)-N-propylamino]-1,2,3,4-tetrahydro-5-hydroxynaphthalin (Verfahren a)

4 g 2-n-Propylamino-1,2,3,4-tetrahydro-5-hydroxynaphthalin werden in 100 ml Dimethylformamid gelöst und 3,8 ml N-Äthyl-N,N-diisopropylamin gefolgt von 2,5 g 5-Bromvaleriansäurenitril zugefügt. Die Lösung wird während 2 Tagen bei 60° gerührt und anschließend das Lösungsmittel am Hochvakuum entfernt. Den Rückstand extrahiert man mit aq. 1n-Natriumbikarbonat-Lösung/Methylenchlorid, die dabei erhaltene organische Phase wird getrocknet (Natriumsulfat), eingeengt und der so erhaltene Rückstand am Kieselgel mit Methylenchlorid (10%ig gesättigt mit Ammoniak)/Methanol = 95/5 chromatographiert. Man erhält das Produkt als amorphen Festkörper, der ins kristalline Hydrochlorid überführt wird (Smp. = 156—58°/Methanol-Äther).

## Beispiel 2

### 1,2,3,4-Tetrahydro-5-hydroxy-2-[N-(3-methylthiopropyl)-N-propylamino]naphthalin (Verfahren b)

1,0 g Natriumhydrid in 40 ml Dimethylformamid wird bei 0° mit 3,3 ml Äthylmercaptan versetzt. Darauf werden 4,5 g 1,2,3,4-Tetrahydro-5-methoxy-2-[N-(3-methylthiopropyl)-N-propylamino]naphthalin in 30 ml Dimethylformamid zugegeben. Dieses Reaktionsgemisch wird 20 Stunden bei 120° gerührt, anschließend abgekühlt, eingedampft und zwischen Äther in 2N Salzsäure verteilt. Die wäßrige Phase wird auf pH 11 bei 5° gestellt und mit Methylenchlorid extrahiert. Nach Trocknen, Abfiltrieren und Eindampfen der organischen Phase erhält man die rohe Titelverbindung, die in das Hydrogenfumarat in Isopropanol/Pentan umgewandelt wird. Smp.: Sintern ab 70° (aus Äthanol/Äther).

Das Ausgangsmaterial kann wie folgt hergestellt werden:

a) 1,2,3,4-Tetrahydro-2-[N-(3-hydroxypropyl)-N-propylamino]-5-methoxynaphthalin

185,4 g 1,2,3,4-Tetrahydro-5-methoxy-2-(propylamino)naphthalin, 236,2 g 3-Jodpropanol, 350,3 g Pottasche und 2 Liter Aceton werden 20 Stunden am Rückfluß gerührt. Man fügt noch 35,8 g 3-Jodpropanol dazu und erwärmt noch 8 Stunden am Rückfluß. Nach Abfiltrieren und Eindampfen wird der Rückstand zwischen 1N Natriumthiosulfat und Methylenchlorid verteilt. Die organische Phase wird über Natriumsulfat getrocknet und eingedampft. Die so erhaltene rohe Titelverbindung wird in das Naphthalin-1,5-disulfonat übergeführt. Smp.: 99 – 101° (Zers.).

b) 2-[N-(3-Chloropropyl)-N-propylamino]-1,2,3,4-tetrahydro-5-methoxynaphthalin

Zu 190 g 1,2,3,4-Tetrahydro-2-[N-(3-hydroxypropyl)-N-propylamino]-5-methoxynaphthalin (Base) in 1,5 Liter Chloroform werden 185,8 g Thionylchlorid zugetropft. Das sich aufgewärmte Reaktionsgemisch wird noch 1 Stunde am Rückfluß erhitzt. Nach Eindampfen wird es zwichen Methylenchlorid und 2N Natronlauge verteilt. Die organische Phase wird mit Wasser gewaschen, über Natriumsulfat getrocknet, filtriert und eingedampft. Die so erhaltene rohe Titelverbindung wird in das Naphthalin-1,5-disulfonat übergeführt. Smp.: 239 – 242° (Zers.).

c) 1,2,3,4-Tetrahydro-5-methoxy-2-[N-(3-methylthiopropyl)-N-propylamino]naphthalin

2,35 g Natrium werden mit 50 ml abs. Äthanol versetzt. Sobald das Natrium sich aufgelöst hat, werden 4,8 g Methylmercaptan in 60 ml Äthanol zugetropft, und es wird auf 45° erwärmt. Bei dieser Temperatur wird dann eine Lösung von 19,7 g 2-[N-(3-Chlorpropyl)-N-propylamino]-1,2,3,4-tetrahydro-5-methoxynaphthalin in 120 ml abs. Äthanol zugetropft. Es wird anschließend 1 Stunde bei 50° gerührt, dann eingedampft. Der Rückstand wird zwischen Äther und Wasser verteilt. Die Ätherphase wird über Pottasche getrocknet, filtriert und eingedampft. Die als öliger Rückstand erhaltene Titelverbindung wird direkt weiterverarbeitet.

Analog zu den Beispielen 1 und 2 wurden aus den entsprechenden Ausgangsverbindungen auch folgende Verbindungen der Formel I [R = H und A = $(CH_2)_n$] hergestellt:

| Beispiel | $R_2$ | n | $R_3$ | Salzform | Smp. |
|---|---|---|---|---|---|
| 3 | $C_3H_7$[6]) | 3 | CN | HCl | 194–197° |
| 4 | $C_3H_7$ | 2 | $NHSO_2N(C_2H_5)_2$ | HCl | 220° |
| 5 | $C_2H_5$ | 3 | CN | HCl | 237–239° |
| 6 | $C_2H_5$ | 3 | $COOCH_3$ | HCl | 160–163° |
| 7 | $C_3H_7$ | 2 | CN | HCl | 203° |
| 8 | $C_2H_5$ | 3 | $CONHCH_3$ | HCl | 179–182° |
| 9 | $C_3H_7$ | 3 | $OCH_3$ | (Base) | 127–128,5° |
| 10 | $C_3H_7$ | 3 | OH | (Base) | 129–130,5° |
| 11 | $C_3H_7$ | 3 | F | HCl | 171–174° |
| 12 | $C_3H_7$ | 3 | $CH{=}CH_2$ | HCl | 148–151° |
| 13 | $C_3H_7$ | 3 | $N_3$ | HCl | 126–128° |
| 14 | $C_3H_7$ | 3 | $SO_2CH_3$ | HCl | 196–200° |
| 15 | $C_3H_7$ | 3 | $SOCH_3$ | (Base) | Öl[1]) |
| 16 | $C_3H_7$ | 3 | Cl | Naphthalin-1,5-disulfonat[7]) | 218–220° |
| 17 | $C_3H_7$ | 3 | $OOCNH_2$ | Naphthalin-1,5-disulfonat[7]) | 156–161° |
| 18 | $C_3H_7$ | 3 | $OOCCH_3$ | (Base) | Öl[2]) |
| 19 | $C_3H_7$ | 3 | $NHCOOC_2H_5$ | HCl | 123° (Zersetzung) |
| 20 | $C_3H_7$ | 3 | $COOCH_3$ | HCl | 159–161° |
| 21 | $C_3H_7$ | 3 | $NHCON(C_2H_5)_2$ | Hydrogenpamoat | 150° (Zersetzung) |
| 22 | $C_3H_7$ | 3 | CN | HCl | 187–188°[3]) |
| 23 | $C_3H_7$ | 3 | CN | HCl | 187–188°[4]) |
| 24 | $C_3H_7$ | 3 | $NHCONHC(CH_3)_3$ | (Base) | Schaum[5]) |
| 25 | $C_3H_7$ | 3 | $NHSO_2NH_2$ | Fumarat | 203–205° (Zers.) |
| 26 | $C_3H_7$ | 3 | $NHSO_2N(CH_3)_2$ | HCl | 161–163° (Zers.) |
| 27 | $C_3H_7$ | 3 | $CONHCH_3$ | Naphthalin-1,5-disulfonat[7]) | 226–229° |
| 28 | $C_3H_7$ | 3 | $NHCONH_2$ | Hydrogenfumarat | 80° (Zersetzung) |
| 29 | $C_3H_7$ | 3 | $NHCOCH_3$ | Naphthalin-1,5-disulfonat[7]) | 148–158° |
| 30 | $CH_3$ | 3 | CN | Naphthalin-1,5-disulfonat[7]) | 230–232° |
| 31 | $C_3H_7$ | 3 | $SCH_3$ | Hydrogenfumarat | 70° (Zersetzung)[8]) |
| 32 | $C_3H_7$ | 3 | $SCH_3$ | Hydrogenfumarat | 70° (Zersetzung)[9]) |

1) NMR ($CDCl_3$) $SOCH_3$: 2,59 $\delta$ (S)—$CH_2$—$C\underline{H_3}$: 0,88 $\delta$ (T)

2) NMR ($CDCl_3$) $C\underline{H_2}OOCCH_3$: 4,13 $\delta$ (T)—OOC—$CH_3$: 2,03 $\delta$ (S)

3) (+)-Antipode der Verbindung des Beispiels 3 $[\alpha]_D^{20}$ = +63° c = 1 (Hydrochlorid in Methanol)

4) (−)-Antipode der Verbindung des Beispiels 3 $[\alpha]_D^{20}$ = −63° c = 1 (Hydrochlorid in Methanol)

Diese Verbindung kann z. B. wie folgt hergestellt werden:

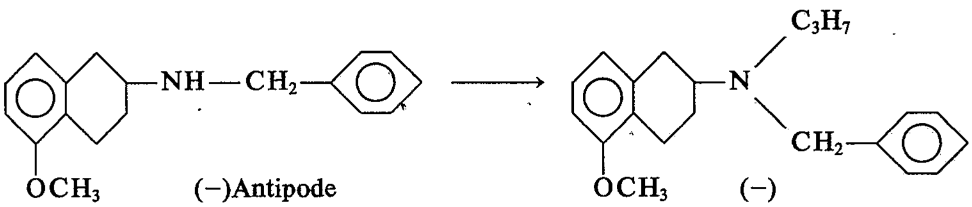

(−)Antipode

(−)

(bekannt aus J. Med. Chem. 19, 547 (1976)

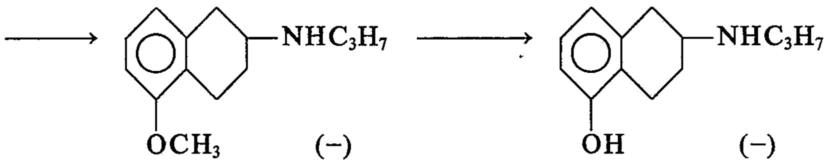

(−)

(−)

Verbindung des Beispiels 23

[5]) NMR ($CDCl_3$) 3 aromat. H: 6,5−7,0 δ (M), 2 NH: 5,3−6,5 δ (breit)
9 tert. Bu-H: 1,38 δ (S), $CH_2$—$C\underline{H_3}$: 0,88 δ (T)

[6]) In dieser Tabelle steht $C_3H_7$ immer für n-$C_3H_7$.

[7]) Diese Salze sind aus 2 Mol Base und 1 Mol Naphthalin-1,5-disulfonsäure zusammengestellt.

[8]) (+)Antipode der Verbindung des Beispiels 2 $[\alpha]_D^{20}$ : +48,6°
(c = 1, Hydrogenfumarat in Methanol)

[9]) (−)Antipode der Verbindung des Beispiels 2 $[\alpha]_D^{20}$ : −48,6°
(c = 1, Hydrogenfumarat in Methanol)

## Beispiel 33

### 2-[N-Äthyl-N-(3-methoxycarbonylpropyl)amino]-1,2,3,4-tetrahydro-5-acetoxynaphthalin (Verfahren c)

1,7 g 2-[N-Äthyl-N-(3-methoxycarbonylpropyl)amino]-1,2,3,4-tetrahydro-5-hydroxynaphthalin werden in 15 ml Trifluoressigsäure gelöst und die Lösung unter Rühren bei Zimmertemperatur tropfenweise mit 1,5 ml Acetylbromid versetzt. Dünnschichtchromatographische Kontrolle nach 1,5 h Rühren zeigt einen praktisch vollständigen Umsatz. Die Lösung wird zur Trockene eingedampft, der Rückstand am Hochvakuum getrocknet und anschließend mit 1 n Natriumkarbonat-Lösung/Methylenchlorid extrahiert. Die organische Phase wird getrocknet, eingeengt, in wenig Aceton aufgenommen und mit 1 Äquivalent einer gesättigten Lösung von Fumarsäure in Aceton versetzt. Das Hydrogenfumarat der Titelverbindung wird aus der eingeengten Acetonlösung, nach sorgfältiger Trocknung am Hochvakuum als amorpher Schaum isoliert.
Analog zu Beispiel 33 wurden auch folgende Verbindungen der Formel I hergestellt:

## Beispiel 34

R = $CH_3CO$    $R_2$ = n-$C_3H_7$    A = $(CH_2)_3$

$R_3$ = CN

Smp. des Hydrogenmalinats: 128−129°

Beispiel 35

$R = C_6H_5—CO$ $R_2 = n\text{-}C_3H_7$ $A = (CH_2)_3$

$R_3 = CN$

Öl—NMR ($CDCl_3$):

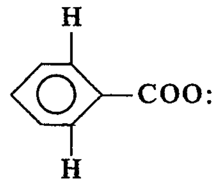

: 8,23 $\delta$ (M)

$CH_2CN$: 2,40 $\delta$ (T)

$C-CH_3$: 0,90 $\delta$ (T)

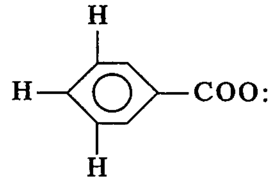

: 7,52 $\delta$ (M)

**Patentansprüche für die Vertragsstaaten: BE, CH, DE, FR, GB, IT, LI, LU, NL, SE**

1. Verbindungen der Formel I

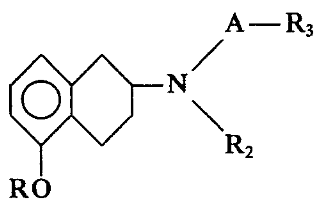

(I)

worin

| | |
|---|---|
| R | für Wasserstoff oder eine $R_1CO$-Gruppe |
| $R_1CO$ | für den unter physiologischen Bedingungen durch Hydrolyse abspaltbaren Acylrest einer Säure |
| $R_2$ | für Alkyl mit 1 bis 4 C-Atomen |
| A | für Alkylen mit 1 bis 5 C-Atomen |
| $R_3$ | für Halogen, eine freie oder veresterte OH-Gruppe, SH, $N_3$, CN, $COR_4$, $NH-X-R_5$, Y-Alkyl mit 1 bis 4 C-Atomen, Y-Phenylalkyl mit 7 bis 10 C-Atomen, Y-Phenyl oder Alkenyl oder Alkinyl mit 2 bis 4 C-Atomen |
| $R_4$ | für Alkoxy mit 1 bis 4 C-Atomen oder 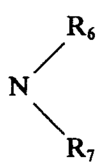 |
| $R_5$ | für Alkyl mit 1 bis 3 C-Atomen, gegebenenfalls durch Alkyl mit 1 bis 4 C-Atomen, Alkoxy mit 1 bis 4 C-Atomen, Fluor, Chlor oder Brom mono- oder disubstituiertes Phenyl, $NR_6R_7$ oder, falls X CO bedeutet, zusätzlich auch für Alkoxy mit 1 bis 4 C-Atomen |
| $R_6$ und $R_7$ | unabhängig voneinander für Wasserstoff oder Alkyl mit 1 bis 4 C-Atomen |
| X | für CO oder $SO_2$ und |
| Y | für O, S, SO oder $SO_2$ |

stehen, in Form der freien Base oder der Säureadditionssalze.

2. Verbindungen der Formel Ia

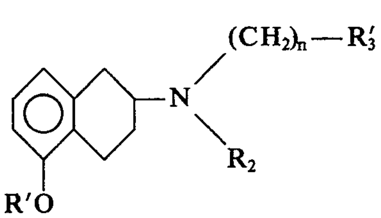

(Ia)

worin

R' für Wasserstoff oder eine $R_1'$ CO-Gruppe
$R_2$ für Alkyl mit 1 bis 4 C-Atomen,
$R_3'$ für eine CN-, $COR_4$-,

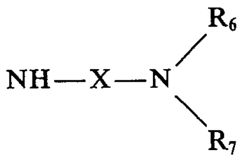

oder $NHCOR_5'$-Gruppe
$R_1'$ für Wasserstoff, Alkyl mit 1 bis 19 C-Atomen, Phenylalkyl mit 7 bis 10 C-Atomen oder gegebenenfalls durch Alkyl mit 1 bis 4 C-Atomen, Alkoxy mit 1 bis 4 C-Atomen, Fluor, Chlor oder Brom mono- oder disubstituiertes Phenyl,
$R_4$ für Alkoxy mit 1 bis 4 C-Atomen oder

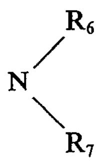

$R_6$ und $R_7$ unabhängig voneinander für Wasserstoff oder Alkyl mit 1 bis 4 C-Atomen,
$R_5'$ für Alkyl mit 1 bis 3 C-Atomen,
X für CO oder $SO_2$
n für eine ganze Zahl von 2 bis 5

stehen, in Form der freien Base oder der Säureadditionssalze.

3. Verbindungen der Formel Ib

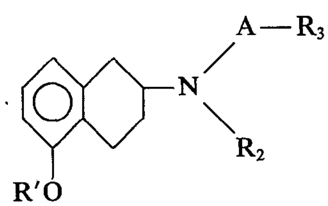

(Ib)

worin

R' und $R_2$ die gemäß Anspruch 2 angegebenen Bedeutungen haben
A für Alkylen mit 1 bis 5 C-Atomen
$R_3$ für Halogen, eine freie oder veresterte OH-Gruppe, SH, $N_3$, $COR_4$, $NH-X-R_5$, Y-Alkyl mit 1 bis 4 C-Atomen, Y-Phenylalkyl mit 7 bis 10 C-Atomen, Y-Phenyl, Alkenyl oder Alkinyl mit 2 bis 4 C-Atomen
$R_4$ für Alkoxy mit 1 bis 4 C-Atomen oder

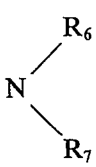

$R_5$ für Alkyl mit 1 bis 3 C-Atomen, gegebenenfalls durch Alkyl mit 1 bis 4 C-Atomen, Alkoxy mit 1 bis 4 C-Atomen, Fluor, Chlor oder Brom mono- oder disubstituiertes Phenyl, $NR_6R_7$ oder, falls X CO bedeutet, zusätzlich auch für Alkoxy mit 1 bis 4 C-Atomen
$R_6$ und $R_7$ unabhängig voneinander für Wasserstoff oder Alkyl mit 1 bis 4 C-Atomen
X für CO oder $SO_2$ und
Y für O, S, SO oder $SO_2$

stehen, mit der Maßgabe, daß, falls A für $(CH_2)_n$, worin n 2 bis 5 bedeutet, steht, $R_3$ nicht CN, $COR_4$,

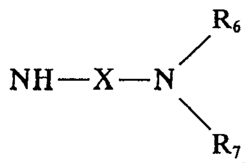

oder $NHCOR_5'$, worin $R_5'$ Alkyl mit 1 bis 3 C-Atomen bedeutet, stehen kann, in Form der freien Base oder der Säureadditionssalze.

4. Eine Verbindung gemäß Anspruch 1, 2 oder 3, worin $R_2$ Propyl ist.

5. Eine Verbindung gemäß Anspruch 1 oder 3, worin A Propylen ist.

6. Eine Verbindung gemäß Anspruch 1 oder 3, worin $R_3$ CN, S Alkyl mit 1 bis 4 C-Atomen, $NHSO_2$, $NR_6R_7$, OH oder F ist.

7. Cyanopropyl)-N-propylamino]-1,2,3,4-tetrahydro-5-hydroxy-naphthalin in Form der freien Base oder der Säureadditionssalze.

8. Die Verbindungen des Anspruchs 7 als (−)-Antipode.

9. 1,2,3,4-Tetrahydro-5-hydroxy-2-[N-(3-methylthiopropyl)-N-propyl-amino]naphthalin in Form der freien Base oder der Säureadditionssalze.

10. Verbindungen des Anspruchs 9 als (−)-Antipode.

11. 1,2,3,4-Tetrahydro-5-hydroxy-2-[N-(3-fluorpropyl)-N-propylamino]naphthalin oder 1,2,3,4-Tetrahydro-5-hydroxy-2-[N-3-chlorpropyl)-N-propylamino]-naphthalin in Form der freien Base oder der Säureadditionssalze.

12. Verfahren zur Herstellung der Verbindungen gemäß Anspruch 1 dadurch gekennzeichnet, daß man

a) zur Herstellung einer Verbindung der Formel I, worin der Rest R für Wasserstoff steht, in eine Verbindung der Formel II

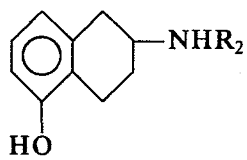

(II)

den Rest $R_3-A$ einführt,

b) zur Herstellung einer Verbindung der Formel I, worin der Rest R für Wasserstoff steht, in einer Verbindung der Formel IV

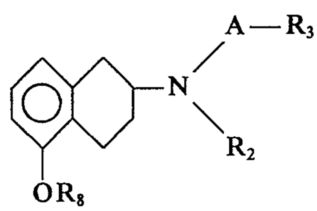

(IV)

worin $R_8$ eine Hydroxyschutzgruppe bedeutet, den Rest $R_8$ abspaltet

c) zur Herstellung einer Verbindung der Formel I, worin der Rest R für die $R_1CO$-Gruppe steht, eine Verbindung der Formel I, worin R für Wasserstoff steht mit $R_1COOH$ oder einem reaktionsfähigen Derivat dieser Carbonsäure acyliert

und die erhaltenen Verbindungen der Formel I in Form der freien Base oder der Säureadditionssalze isoliert.

13. Pharmazeutische Präparate enthaltend mindestens eine Verbindung gemäß einem der Ansprüche 1 bis 11 in Form der Basen oder der pharmazeutisch geeigneten Säureadditionssalze.

14. Eine Verbindung gemäß einer der Ansprüche 1 bis 11 in Form der freien Base oder der pharmazeutisch geeigneten Säureadditionssalze zur Verwendung als Pharmazeutikum.

15. Eine Verbindung gemäß einem der Ansprüche 1 bis 11 in Form der freien Base oder der pharmazeutisch geeigneten Säureadditionssalze zur Verwendung als Prolaktinsekretionshemmer, bei der Behandlung von Herz-Kreislauferkrankungen oder bei Parkinsonismus.

**Patentansprüche für den Vertragsstaat AT**

1. Verfahren zur Herstellung von Verbindungen der Formel I

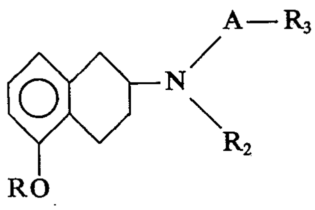

(I)

worin

| | |
|---|---|
| R | für Wasserstoff oder eine $R_1CO$-Gruppe |
| $R_1CO$ | für den unter physiologischen Bedingungen durch Hydrolyse abspaltbaren Acylrest einer Säure |
| $R_2$ | für Alkyl mit 1 bis 4 C-Atomen |
| A | für Alkylen mit 1 bis 5 C-Atomen |
| $R_3$ | für Halogen, eine freie oder veresterte OH-Gruppe, SH, $N_3$, CN, $COR_4$, $NH-X-R_5$, Y-Alkyl mit 1 bis 4 C-Atomen, Y-Phenylalkyl mit 7 bis 10 C-Atomen, Y-Phenyl oder Alkenyl oder Alkinyl mit 2 bis 4 C-Atomen |
| $R_4$ | für Alkoxy mit 1 bis 4 C-Atomen oder |

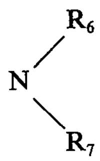

| | |
|---|---|
| $R_5$ | für Alkyl mit 1 bis 3 C-Atomen, gegebenenfalls durch Alkyl mit 1 bis 4 C-Atomen, Alkoxy mit 1 bis 4 C-Atomen, Fluor, Chlor oder Brom mono- oder disubstituiertes Phenyl, $NR_6R_7$ oder, falls X CO bedeutet, zusätzlich auch für Alkoxy mit 1 bis 4 C-Atomen |
| $R_6$ und $R_7$ | unabhängig voneinander für Wasserstoff oder Alkyl mit 1 bis 4 C-Atomen |
| X | für CO oder $SO_2$ und |
| Y | für O, S, SO oder $SO_2$ |

stehen, in Form der freien Base oder der Säureadditionssalze, dadurch gekennzeichnet, daß man

a) zur Herstellung einer Verbindung der Formel 1, worin der Rest R für Wasserstoff steht, in eine Verbindung der Formel II

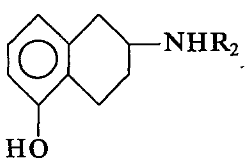

(II)

den Rest $R_3-A$ einführt,

b) zur Herstellung einer Verbindung der Formel I, worin der Rest R für Wasserstoff steht, in einer Verbindung der Formel IV

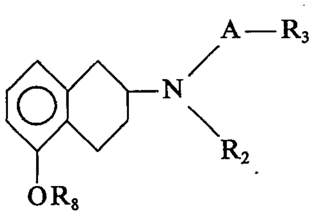

(IV)

worin $R_8$ eine Hydroxyschutzgruppe bedeutet, den Rest $R_8$ abspaltet

c) zur Herstellung einer Verbindung der Formel I, worin der Rest R für die $R_1CO$-Gruppe steht, eine Verbindung der Formel I, worin R für Wasserstoff steht mit $R_1COOH$ oder einem reaktionsfähigen Derivat dieser Carbonsäure acyliert

und die erhaltenen Verbindungen der Formel I in Form der freien Base oder der Säureadditionssalze isoliert.

2. Verfahren gemäß Anspruch 1, dadurch gekennzeichnet, daß Verbindungen der Formel Ia

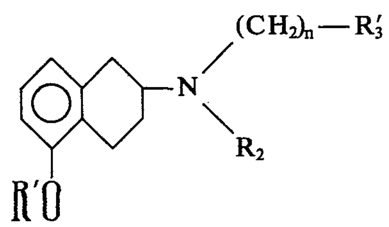

(Ia)

worin

| | |
|---|---|
| R' | für Wasserstoff oder eine $R_1$' CO-Gruppe |
| $R_2$ | für Alkyl mit 1 bis 4 C-Atomen, |
| $R_3$' | für eine CN-, $COR_4$-, |

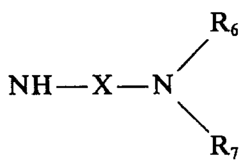

oder $NHCOR_5$'-Gruppe

| | |
|---|---|
| $R_1$' | für Wasserstoff, Alkyl mit 1 bis 19 C-Atomen, Phenylalkyl mit 7 bis 10 C-Atomen oder gegebenenfalls durch Alkyl mit 1 bis 4 C-Atomen, Alkoxy mit 1 bis 4 C-Atomen, Fluor, Chlor oder Brom mono- oder disubstituiertes Phenyl, |
| $R_5$ | für Alkoxy mit 1 bis 4 C-Atomen oder |

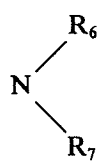

| | |
|---|---|
| $R_6$ und $R_7$ | unabhängig voneinander für Wasserstoff oder Alkyl mit 1 bis 4 C-Atomen, |
| $R_5$' | für Alkyl mit 1 bis 3 C-Atomen, |
| X | für CO oder $SO_2$ |
| n | für eine ganze Zahl von 2 bis 5 |

stehen, in Form der freien Base oder der Säureadditionssalze isoliert werden.

3. Verfahren gemäß Anspruch 1, dadurch gekennzeichnet, daß Verbindungen der Formel Ib

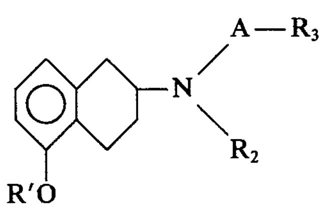

(Ib)

worin

| | |
|---|---|
| R' und $R_2$ | die für die Formel Ia angegebenen Bedeutungen haben |
| A | für Alkylen mit 1 bis 5 C-Atomen |
| $R_3$ | für Halogen, eine freie oder veresterte OH-Gruppe, SH, $N_3$, CN, $COR_4$, $NH-X-R_5$, Y-Alkyl mit 1 bis 4 C-Atomen, Y-Phenylalkyl mit 7 bis 10 C-Atomen, Y-Phenyl, Alkenyl oder Alkinyl mit 2 bis 2 C-Atomen |
| $R_4$ | für Alkoxy mit 1 bis 4 C-Atomen oder |

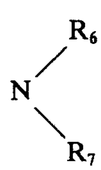

| | |
|---|---|
| $R_5$ | für Alkyl mit 1 bis 3 C-Atomen, gegebenenfalls durch Alkyl mit 1 bis 4 C-Atomen, Alkoxy mit 1 bis 4 C-Atomen, Alkoxy mit 1 bis 4 C-Atomen, Fluor, Chlor oder Brom mono- oder |

disubstituiertes Phenyl, $NR_6R_7$ oder, falls X CO bedeutet, zusätzlich auch für Alkoxy mit 1 bis 4 C-Atomen

$R_6$ und $R_7$ unabhängig voneinander für Wasserstoff oder Alkyl mit 1 bis 4 C-Atomen

X für CO oder $SO_2$ und

Y für O, S, SO oder $SO_2$

stehen, mit der Maßgabe, daß, falls A $(CH_2)_n$, worin n 2 bis 5 bedeutet, steht, $R_3$ nicht CN, $COR_4$,

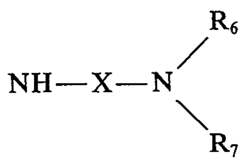

oder $NHCOR_5'$, worin $R_5'$ Alkyl mit 1 bis 3 C-Atomen bedeutet, stehen kann in Form der freien Base oder der Säureadditionssalze isoliert werden.

4. Verfahren gemäß Anspruch 1, 2 oder 3 zur Herstellung von Verbindungen, worin $R_2$ Propyl ist.

5. Verfahren gemäß Anspruch 1 oder 3, zur Herstellung von Verbindungen, worin A Propylen ist.

6. Verfahren gemäß Anspruch 1 oder 3, zur Herstellung von Verbindungen, worin $R_3$ CN, S Alkyl mit 1 bis 4 C-Atomen, $NHSO_2$, $NR_6R_7$, OH oder F ist.

7. Verfahren gemäß Anspruch 1, dadurch gekennzeichnet, daß 2-[N-(3-Cyanopropyl)-N-propylamino]-1,2,3,4-tetrahydro-5-hydroxy-naphthalin in Form der freien Base oder der Säureadditionssalze isoliert wird.

8. Verfahren gemäß Anspruch 7, zur Herstellung der Verbindungen als (−)-Antipode.

9. Verfahren gemäß Anspruch 1, dadurch gekennzeichnet, daß 1,2,3,4-Tetrahydro-5-hydroxy-2-[N-(3-methylthiopropyl)-N-propyl-amino)naphthalin in Form der freien Base oder der Säureadditionssalze isoliert wird.

10. Verfahren gemäß Anspruch 9, zur Herstellung der Verbindungen als (−)-Antipode.

11. Verfahren gemäß Anspruch 1, zur Herstellung von 1,2,3,4-Tetrahydro-5-hydroxy-2[N-(3-fluor-propyl)-N-propylamino]-naphthalin oder 1,2,3,4-Tetrahydro-5-hydroxy-2-[N-3-chlorpropyl)-N-propylamino]-naphthalin in Form der freien Base oder der Säureadditionssalze.

**Claims for the contracting states: BE, CH, DE, FR, GB, IT, LI, LU, NL, SE**

1. Compounds of formula I

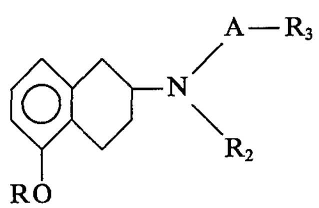

(I)

wherein

R is hydrogen or a $R_1CO$-radical

$R_1CO$ is an acyl radical of an acid, which radical is under physiological conditions splittable by hydrolysis.

$R_2$ is alkyl with 1 to 4 carbon atoms

A is alkylene with 1 to 5 carbon atoms

$R_3$ is halogen, a free or esterified OH group. SH, $N_3$, CN, $COR_4$, $NH-X-R_5$, Y-alkyl with 1 to 4 carbon atoms, Y-phenylalkyl with 7 to 10 carbon atoms, Y-phenyl or alkenyl or alkynyl with 2 to 4 carbon atoms

$R_4$ is alkoxy with 1 to 4 carbon atoms or

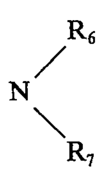

$R_5$ is alkyl with 1 to 3 carbon atoms, phenyl, optionally mono- or disubstituted by alkyl with 1 to 4 carbon atoms, alkoxy with 1 to 4 carbon atoms, fluorine, chlorine or bromine, $NR_6R_7$ or, if X

is CO, additionally is also alkoxy with 1 to 4 carbon atoms
$R_6$ and $R_7$ independently are hydrogen or alkyl with 1 to 4 carbon atoms
X is CO or $SO_2$ and
Y is O, S, SO or $SO_2$

in form of the free base or of the acid addition salts.

2. Compounds of formula Ia

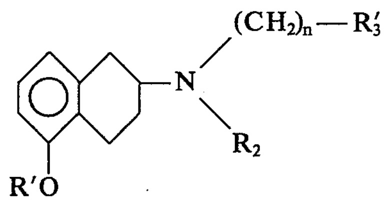

(Ia)

wherein

R′ is hydrogen or a $R_1$′CO radical
$R_2$ is alkyl with 1 to 4 carbon atoms
$R_3$′ is a CN, $COR_4$,

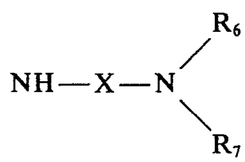

or $NHCOR_5$′ radical
$R_1$′ is hydrogen, alkyl with 1 to 19 carbon atoms, phenylalkyl with 7 to 10 carbon atoms or phenyl, optionally mono- or disubstituted by alkyl with 1 to 4 carbon atoms, alkoxy with 1 to 4 carbon atoms, fluorine, chlorine or bromine,
$R_4$ is alkoxy with 1 to 4 carbon atoms or

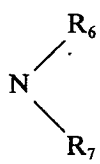

$R_6$ and $R_7$ are independently hydrogen or alkyl with 1 to 4 carbon atoms
$R_5$′ is alkyl with 1 to 3 carbon atoms
X is CO or $SO_2$
n is a whole number from 2 to 5

in form of the freee base or of the acid addition salts.

3. Compounds of the formula Ib

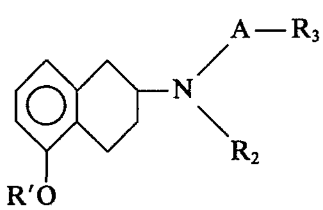

(Ib)

wherein

R′ and $R_2$ are as indicated in claim 2
A is alkylene with 1 to 5 carbon atoms
$R_3$ is halogen, a free or esterified OH-group, SH, $N_3$, CN, $COR_4$, $NHXR_5$, Y-alkyl with 1 to 4 carbon atoms, Y-phenylalkyl with 7 to 10 carbon atoms, Y-phenyl, alkenyl or alkynyl with 2 to 4 carbon atoms
$R_4$ ist alkoxy with 1 to 4 carbon atoms or

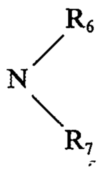

| | |
|---|---|
| $R_5$ | is alkyl with 1 to 3 carbon atoms, optionally by alkyl with 1 to 4 carbon atoms, alkoxy with 1 to 4 carbon atoms, fluorine, chlorine or bromine mono-or disubstituted phenyl, $NR_6R_7$ or, if X is CO, additionally is also alkoxy with 1 to 4 carbon atoms. |
| $R_6$ and $R_7$ | are independently hydrogen or alkyl with 1 to 4 carbon atoms |
| X | is CO or $SO_2$ and |
| Y | is O, S, SO or $SO_2$ |

with the proviso that, if A is $(CH_2)_n$ in which n is 2 to 5 $R_3$ is not CN, $COR_4$,

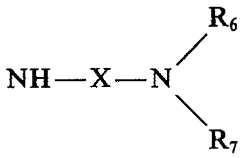

or $NHCOR_5'$, wherein $R_5'$ is alkyl with 1 to 3 carbon atoms, in form of the free base or of the acid addition salts.

4. A compound according to claim 1, 2 or 3 wherein $R_2$ is propyl.

5. A compound according to claim 1 or 3, wherein A is propylene.

6. A compound according to claim 1 or 3, wherein $R_3$ is CN, S-alkyl with 1 to 4 carbon atoms, $NHSO_2NR_6R_7$, OH or F.

7. 2-[N-(3-cyanopropyl)-N-propylamino]1,2,3,4-tetrahydro-5-hydroxy-naphthalene in the form of the free base or of the acid addition salts.

8. The compounds of claim 7 as (−)-antipode.

9. 1,2,3,4-Tetrahydro-5-hydroxy-2-[N-(3-methylthiopropyl)-N-propyl-amino]naphthalene in form of the free base or of the acid addition salts.

10. The compounds of claim 9 as (−)-antipode.

11. 1,2,3,4-Tetrahydro-5-hydroxy-2-[N-(3-fluoropropyl)-N-propylamino]naphthalene or 1,2,3,4-tetrahydro-5-hydroxy-2-[N-(3-chloropropyl)-N-propylamino]naphthalene in form of the free base or of the acid addition salts.

12. Process for the production of compounds according to claim 1 characterised in that

a) for the production of a compound of formula I wherein R is hydrogen, the radical $R_3-A$ is introduced into a compound of formula II ·

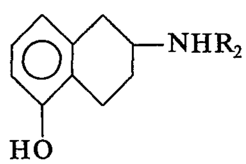

(II)

b) for the production of a compound of formula I, wherein the radical R is hydrogen, the radical $R_8$ is split off, in a compound of formula IV

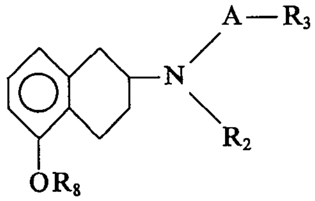

(IV)

wherein $R_8$ is a hydroxy protecting group,

c) for the production of a compound of formula I, wherein the radical R is the $R_1CO$-group, a compound of formula I, wherein R is hydrogen, acylated with $R_1COOH$ or with a reactive derivative of this carboxylic acid,

and the obtained compounds of formula I are isolated in the form of the free base or of the acid addition salts.

13. Pharmaceutical compositions containing at least one compound according to one of the claims 1 to 11 in the form of the base or of the pharmaceutically acceptable acid addition salts.

14. A compound according to one of the claims 1 to 11 in the form of the free base or of the pharmaceitically acceptable acid addition salts for use as a pharmaceutical.

15. A compound according to one of the claims 1 to 11 in the form of the free base or of the pharmaceutically acceptable acid addition salts for use as a prolactin secretion inhibitor, in the treatment of heart circulation illnesses or in Parkinsonism.

**Claims for the contracting state at**

1. Process for the production of compounds of formula I

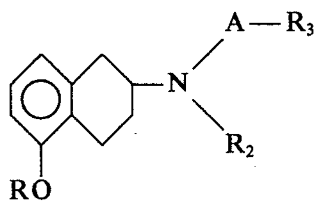

(I)

wherein

R is hydrogen or a $R_1CO$-radical
$R_1CO$ is an acyl radical of an acid, which radical is under physiological conditions splittable by hydrolysis,
$R_2$ is alkyl with 1 to 4 carbon atoms
A is alkylene with 1 to 5 carbon atoms
$R_3$ is halogen, a free or esterified OH group, SH, $N_3$, CN, $COR_4$, $NH-X-R_5$, Y-alkyl with 1 to 4 carbon atoms, Y-phenylalkyl with 7 to 10 carbon atoms, Y-phenyl or alkenyl or alkynyl with 2 to 4 carbon atoms
$R_4$ is alkoxy with 1 to 4 carbon atoms or

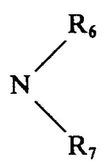

$R_5$ is alkyl with 1 to 3 carbon atoms, optionally by alkyl with 1 to 4 carbon atoms, alkoxy with 1 to 4 carbon atoms, fluorine, chlorine or bromine mono-or disubstituted phenyl, $NR_6R_7$ or, if X is CO, additionally is also alkoxy with 1 to 4 carbon atoms
$R_6$ and $R_7$ independently are hydrogen or alkyl with 1 to 4 carbon atoms
X is CO or $SO_2$ and
Y is O, S, SO or $SO_2$,

in form of the free base or of the acid addition salts characterised in that

a) for the production of a compound of formula I wherein
R ist hydrogen,
in a compound of formula II

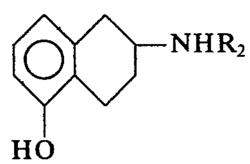

(II)

the radical $R_3-A$ is introduced,

b) for the production of a compound of formula I, wherein
the radical R ist hydrogen,
in a compound of formula IV

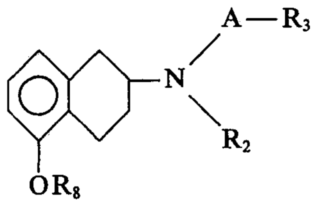

(IV)

wherein $R_8$ is a hydroxy protecting group, the radical $R_8$ is splitted of,

c) for the production of a compound of formula I, wherein the radical R is the $R_1CO$-group, a compound of formula I, wherein R is hydrogen, is acylated with $R_1COOH$ or with a reactive derivative of this carboxylic acid,

and the obtained componds of formula I are isolated in form of the free base or of the acid addition salts.

2. Process according to claim 1, characterised in that compounds of the formula Ia

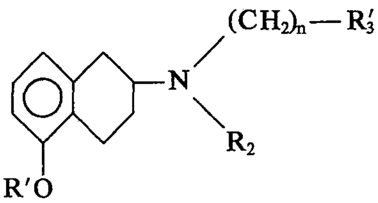

(Ia)

wherein

R' is hydrogen or a $R_1'CO$ radical
$R_2$ is alkyl with 1 to 4 carbon atoms
$R_3'$ is a CN, $COR_4$,

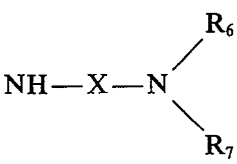

or $NHCOR_5'$ radical
$R_1'$ is hydrogen, alkyl with 1 to 19 carbon atoms, phenylalkyl with 7 to 10 carbon atoms or optionally by alkyl with 1 to 4 carbon atoms, alkoxy with 1 to 4 carbon atoms, fluorine, chlorine or bromine mono- or disubstituted phenyl
$R_4$ is alkoxy with 1 to 4 carbon atoms or

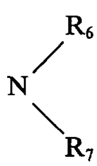

$R_6$ and $R_7$ are independently hydrogen or alkyl with 1 to 4 carbon atoms
$R_5'$ is alkyl with 1 to 3 carbon atoms
X is CO or $SO_2$
n is a whole number of 2 to 5

in form of the free base or of the acid addition salts, are isolated.

3. Process according to claim 1, characterised in that compounds of the formula Ib

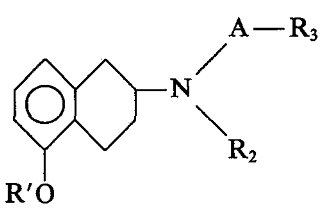

(Ib)

wherein

$R'$ and $R_2$ are as indicated for formula Ia
A is alkylene with 1 to 5 carbon atoms
$R_3$ is halogen, a free or esterified OH-group, SH, $N_3$, CN, $COR_4$, $NHXR_5$, Y-alkyl with 1 to 4 carbon atoms, Y-phenylalkyl with 7 to 10 carbon atoms, Y-phenyl, alkenyl or alkynyl with 2 to 2 carbon atoms
$R_4$ is alkoxy with 1 to 4 carbon atoms or

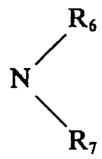

$R_5$ is alkyl with 1 to 3 carbon atoms, optionally by alkyl with 1 to 4 carbon atoms, alkoxy with 1 to 4 carbon atoms, fluorine, chlorine or bromine mono- or disubstituted phenyl, $NR_6R_7$ or, if X is CO, additionally is also alkoxy with 1 to 4 carbon atoms,
$R_6$ and $R_7$ are independently hydrogen or alkyl with 1 to 4 carbon atoms
X is CO or $SO_2$ and
Y is O, S, SO or $SO_2$,

with the proviso that, if A is $(CH_2)_n$ in which n is 2 to 5 $R_3$ is not CN, $COR_4$,

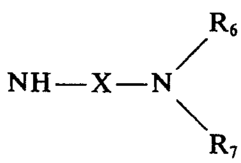

or $NHCOR_5'$, wherein $R_5'$ is alkyl with 1 to 3 carbon atoms, in form of the free base or of the acid addition salts, are isolated.

4. Process according to claim 1, 2 or 3 for the production of compounds in which $R_2$ is propyl.

5. Process according to claim 1 or 3, for the production of compounds in which A is propylene.

6. Process according to claim 1 or 3, for the production of compounds in which $R_3$ is CN, S-alkyl with 1 to 4 carbon atoms, $NHSO_2NR_6R_7$, OH or F.

7. Processe according to claim 1, characterised in that 2-[N-(3-cyano-propyl-N-propylamino]-1,2,3,4-tetrahydro-5-hydroxy-naphthalene is isolated in form of the free base or of the acid addition salts.

8. Process according to claim 7, for the production of the compounds as the (−)-antipode.

9. Process according to claim 1, characterised in that 1,2,3,4-tetrahydro-5-hydroxy-2-[N-(3-methylthiopropyl)-N-propylamino)naphthalene is isolated in form of the free base or of the acid addition salts.

10. Process according to claim 9, for the production of the compounds as the (−)-antipode.

11. Process according to claim 1, for the production of 1,2,3,4-tetrahydro-5-hydroxy-2-[N-(3-fluoropropyl)-N-propylamino]naphthalene or 1,2,3,4-tetrahydro-5-hydroxy-2-[N-(3-chloropropyl)-N-propylamino]naphthalene in the form of the free base or of the acid addition salts.

**Revendications pour les Etats contractants BE, CH, DE, FR, GB, IT, LI, LU, NL, SE**

1. Composés de formule I

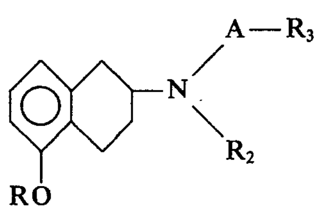

(I)

dans laquelle

R représente l'hydrogène ou un groupe $R_1CO$,
$R_1CO$ représente le reste acyle d'un acide, éliminable par hydrolyse sous des conditions physiologiques,

| | |
|---|---|
| $R_2$ | représente un groupe alkyle contenent de 1 à 4 atomes de carbone, |
| A | représente un groupe alkylène contenant de 1 à 5 atomes de carbone, |
| $R_3$ | représente un halogène, un groupe OH libre ou estérifié, SH, $N_3$, CN, $COR_4$, $NH-X-R_5$, Y-alkyle contenant de 1 à 4 atomes de carbone, Y-phénylalkyle contenant de 7 à 10 atomes de carbone, Y-phényle ou alcényle ou alcynyle contenant de 2 à 4 atomes de carbone, |
| $R_4$ | représente un groupe alcoxy contenant de 1 à 4 atomes de carbone ou 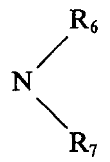 |
| $R_5$ | représente un groupe alkyle contenant de 1 à 3 atomes de carbone, un groupe phényle éventuellement mono- ou disubstitué par alkyle contenant de 1 à 4 atomes de carbone, alcoxy contenant de 1 à 4 atomes de carbone, fluor, chlore ou brome, un groupe $NR_6R_7$ ou, lorsque X signifie CO, également un groupe alcoxy contenant de 1 à 4 atomes de carbone, |
| $R_6$ et $R_7$ | représentent, indépendamment l'un de l'autre, l'hydrogène ou un groupe alkyle contenant de 1 à 4 atomes de carbone, |
| X | représente CO ou $SO_2$ et |
| Y | représente O, S, SO ou $SO_2$, |

sous forme de base libre ou de sels d'addition d'acides.

2. Composés de formule Ia

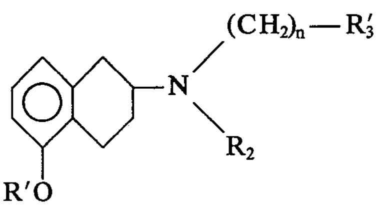

(Ia)

dans laquelle

| | |
|---|---|
| R' | représente l'hydrogène ou un groupe $R_1'CO$, |
| $R_2$ | représente un groupe alkyle contenant de 1 à 4 atomes de carbone, |
| $R_3'$ | représente un groupe CN, $COR_4$, 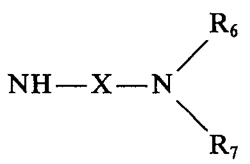 ou $NHCOR_5'$, |
| $R_1'$ | représente l'hydrogène, un groupe alkyle contenent de 1 à 19 atomes de carbone, phénylalkyle contenant de 7 à 10 atomes de carbone ou phényle éventuellement mono- ou disubstitué par alkyle contenant de 1 à 4 atomes de carbone, alcoxy contenant de 1 à 4 atomes de carbone, fluor, chlore ou brome, |
| $R_4$ | représente un groupe alcoxy contenant de 1 à 4 atomes de carbone ou 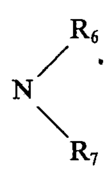 |
| $R_6$ et $R_7$ | représentent, independamment l'un de l'autre, l'hydrogène ou un groupe alkyle contenant de 1 à 4 Atomes de carbone, |
| $R_5'$ | représente un groupe alkyle contenant de 1 à 3 atomes de carbone, |
| X | représente CO ou $SO_2$, |
| n | représente un nombre entier de 2 à 5, |

sous forme de base libre ou de sels d'addition d'acides.

3. Composés de formule Ib

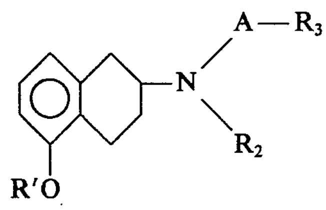

(Ib)

dans laquelle

| | |
|---|---|
| R' et $R_2$ | ont les significations données à la revendication 2, |
| A | représente un groupe alkylène contenant de 1 à 5 atomes de carbone, |
| $R_3$ | représente un halogène, un groupe OH libre ou estérifié, SH, $N_3$, CN, $COR_4$, $NH-X-R_5$, Y-alkyle contenant de 1 à 4 atomes de carbone, Y-phénylalkyle contenant de 7 à 10 atomes de carbone, Y-phényle, alcényle ou alcynyle contenant de 2 à 4 atomes de carbone, |
| $R_4$ | représente un groupe alcoxy contenant de 1 à 4 atomes de carbone ou 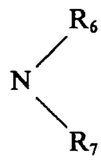 |
| $R_5$ | représente un groupe alkyle contenant de 1 à 3 atomes de carbone, un groupe phényle éventuellement mono- ou disubstitué par alkyle contenant de 1 à 4 atomes de carbone, alcoxy contenant de 1 à 4 atomes de carbone, fluor, chlore ou brome, un groupe $NR_6R_7$ ou, lorsque X signifie CO, également un groupe alcoxy contenant de 1 à 4 atomes de carbone, |
| $R_6$ et $R_7$ | représentent, indépendamment l'un de l'autre, l'hydrogène ou un groupe alkyle contenant de 1 à 4 atomes de carbone, |
| X | représente CO ou $SO_2$ et |
| Y | représente O, S, SO ou $SO_2$, |

avec la condition que, lorsque A représente $(CH_2)_n$ où signifie 2 à 5, $R_3$ ne peut représenter CN, $COR_4$,

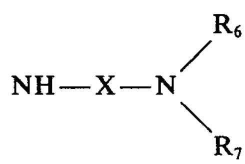

ou $NHCOR_5'$ où $R_5'$ signifie un groupe alkyle contenant de 1 à 3 atomes de carbone, sous forme de base libre ou de sels d'addition d'acides.

4. Un composé selon la revendication 1, 2 ou 3, caractérisé en ce que $R_2$ signifie propyle.

5. Un composé selon la revendication 1 ou 3, caractérisé en ce que A signifie propylène.

6. Un composé selon la revendication 1 ou 3, caractérisé en ce que $R_3$ signifie CN, S-alkyle contenant de 1 à 4 atomes de carbone, $NHSO_2NR_6R_7$, OH ou F.

7. Le 2-[N-(3-cyanopropyl)-N-propylamino]-1,2,3,4-tétrahydro-5-hydroxy-naphtalène sous forme de base libre ou de sels d'addition d'acides.

8. Les composés de la revendication 7 sous forme d'antipode (−).

9. Le 1,2,3,4-tétrahydro-5-hydroxy-2-[N-(3-méthylthiopropyl)-N-propyl-amino]naphtalène sous forme de base libre ou de sels d'addition d'acides.

10. Les composés de la revendication 9 sous forme d'antipode (−).

11. Le 1,2,3,4-tétrahydro-5-hydroxy-2-[N-(3-fluoropropyl)-N-propylamino]naphthalène ou le 1,2,3,4-tétrahydro-5-hydroxy-2-[N-(3-chloropropyl)-N-propylamino]naphtalène sous forme de base libre ou de sels d'addition d'acides.

12. Procédé de préparation des composés selon la revendication 1, caractérisé en ce que

a) pour préparer un composé de formule I, dans laquelle le rest R représente l'hydrogène, on introduit dans un composé de formule II

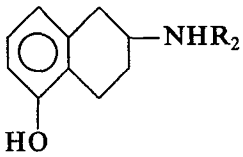

(II)

le reste $R_3-A$,

b) pour préparer un composé de formule I, dans laquelle le reste R représente l'hydrogène, on élimine dans un composé de formule IV

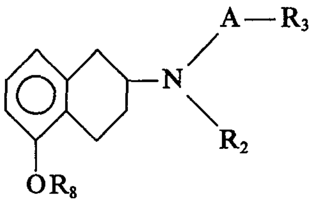

(IV)

dans laquelle $R_8$ signifie un groupe protecteur du groupe hydroxy, le reste $R_8$,

c) pour préparer un composé de formule I, dans laquelle le reste R représente le groupe $R_1CO$, on acyle un composé de formule I, dans laquelle R représente l'hydrogène, avec $R_1COOH$ ou un dérivé réactif de cet acide,

et on isole les composés obtenus de formule I, sous forme de base libre ou de sels d'addition d'acides.

13. Compositions pharmaceutiques, caractérisées en ce qu'elles contiennent au moins un composé selon l'une des revendications 1 à 11, sous forme de base ou de sels d'addition d'acides appropriés du point de vue pharmaceutique.

14. Un composé selon l'une des revendications 1 à 11 sous forme de base libre ou de sels d'addition d'acides appropriés du point de vue pharmaceutique, pour l'utilisation comme médicament.

15. Un composé selon l'une des revendications 1 à 11 sous forme de base libre ou de sels d'addition d'acides appropriés du point de vue pharmaceutique, pour l'utilisation comme inhibiteur de la sécrétion de prolactine, dans le traitement des maladies cardiovasculaires ou dans la maladie de Parkinson.

**Revendications pour l'Etat contractant AT**

1. Procédé de préparation des composés de formule I

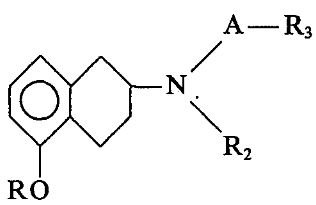

(I)

dans laquelle

R représente l'hydrogène ou un groupe $R_1CO$,

$R_1CO$ représente le reste acyle d'un acide, éliminable par hydrolyse sous des conditions physiologiques,

$R_2$ représente un groupe alkyle contenant de 1 à 4 atomes de carbone,

A représente un groupe alkylène contenant de 1 à 5 atomes de carbone,

$R_3$ représente un halogène, un groupe OH libre ou estérifié, SH, $N_3$, CN, $COR_4$, $NH-X-R_5$, Y-alkyle contenant de 1 à 4 atomes de carbone, Y-phénylalkyle contenant de 7 à 10 atomes de carbone, Y-phényle ou alcényle ou alcynyle contenant de 2 à 4 atomes de carbone,

$R_4$ représente un groupe alcoxy contenant de 1 à 4 atomes de carbone ou

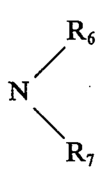

$R_5$ représente un groupe alkyle contenant de 1 à 3 atomes de carbone, un groupe phényle éventuellement mono- ou disubstitué par alkyle contenant de 1 à 4 atomes de carbone, alcoxy contenant de 1 à 4 atomes de carbone, fluor, chlore ou brome, un groupe $NR_6R_7$ ou, lorsque X signifie CO, également un groupe alcoxy contenant de 1 à 4 atomes de carbone,

$R_6$ et $R_7$ représentent, indépendamment l'un de l'autre, l'hydrogène ou un groupe alkyle contenant de 1 à 4 atomes de carbone,

X représente CO ou $SO_2$ et

Y représente O, S, SO ou $SO_2$,

sous forme de base libre ou de sels d'addition d'acides, caractérisé en ce que

a) pour préparer un composé de formule I, dans laquelle le reste R représente l'hydrogène, on introduit dans un composé de formule II

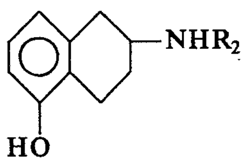

(II)

le reste $R_3-A$,

b) pour préparer un composé de formule I, dans laquelle le reste R représente l'hydrogène, on élimine dans un composé de formule IV

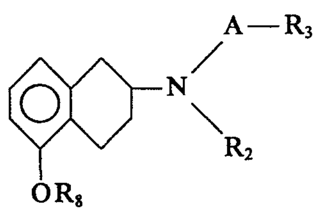

(IV)

dans laquelle $R_8$ signifie un groupe protecteur du groupe hydroxy, le reste $R_8$,

c) pour préparer un composé de formule I, dans laquelle le reste R représente le groupe $R_1CO$, on acyle un composé de formule I, dans laquelle R représente l'hydrogène, avec $R_1COOH$ ou un dérivé réactif de cet acide,

et on isole les composés obtenus de formule I, sous forme de base libre ou de sels d'addition d'acides.

2. Procédé selon la revendication 1, caractérisé en ce qu'on isole des composés de formule Ia

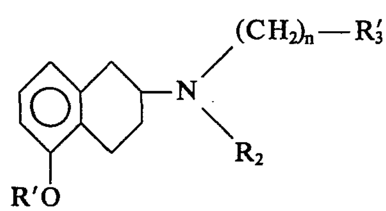

(Ia)

dans laquelle

R' représente l'hydrogène ou un groupe $R_1'CO$,

$R_2$ représente un groupe alkyle contenant de 1 à 4 atomes de carbone,

$R_3'$ représente un groupe CN, $COR_4$,

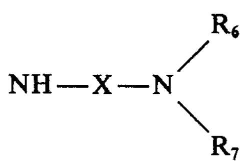

ou $NHCOR_5'$,

$R_1'$ représente l'hydrogène, un groupe alkyle contenant de 1 à 19 atomes de carbone, phénylalkyle contenant de 7 à 10 atomes de carbone ou phényle éventuellement mono- ou disubstitué par alkyle contenant de 1 à 4 atomes de carbone, alcoxy contenant de 1 à 4 atomes de carbone, fluor, chlore ou brome,

$R_4$ représente un groupe alcoxy contenant de 1 à 4 atomes de carbone ou

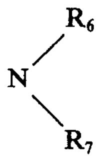

$R_6$ et $R_7$ représentent, indépendamment l'un de l'autre, l'hydrogène ou un groupe alkyle contenant de 1 à 4 atomes de carbone,
$R_5'$ représente un groupe alkyle contenant de 1 à 3 atomes de carbone,
X représente CO ou $SO_2$,
n représente un nombre entier de 2 à 5,

sous forme de base libre ou de sels d'addition d'acides.

3. Procédé selon la revendication 1, caractérisé en ce qu'on isole des composés de formule Ib

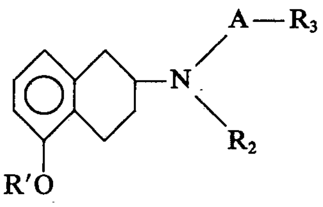

(Ib)

dans laquelle

R' et $R_2$ ont les significations données pour la formule Ia,
A représente un groupe alkylène contenant de 1 à 5 atomes de carbone,
$R_3$ représente un halogène, un groupe OH libre ou estérifié, SH, $N_3$, CN, $COR_4$, $NH-X-R_5$, Y-alkyle contenant de 1 à 4 atomes de carbone, Y-phénylalkyle contenant de 7 à 10 atomes de carbone, Y-phényle, alcényle ou alcynyle contenant de 2 à 2 atomes de carbone,
$R_4$ représente un groupe alcoxy contenant de 1 à 4 atomes de corbone ou

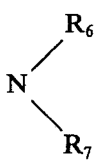

$R_5$ représente un groupe alkyle contenant de 1 à 3 atomes de carbone, un groupe phényle éventuellement mono- ou disubstitué par alkyle contenant de 1 à 4 atomes de carbone, alcoxy contenant de 1 à 4 atomes de carbone, fluor, chlore ou brome, un groupe $NR_6R_7$ ou, lorsque X signifie CO, également un groupe alcoxy contenant de 1 à 4 atomes de carbone,
$R_6$ et $R_7$ représentent, indépendamment l'un de l'autre, l'hydrogène ou un groupe alkyle contenant de 1 à 4 atomes de carbone,
X représente CO ou $SO_2$ et
Y représente O, S, SO ou $SO_2$,

avec la condition que, lorsque A représente $(CH_2)_n$ où n signifie 2 à 5, $R_3$ ne peut représenter CN, $COR_4$,

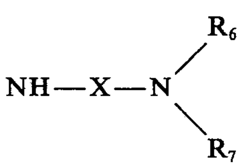

ou $NHCOR_5'$ ou $R_5$ signifie un groupe alkyle contenant de 1 à 3 atomes de carbone, sous forme de base libre ou de sels d'addition d'acides.

4. Procédé selon la revendication 1, 2 ou 3 pour la préparation de composés où $R_2$ signifie propyle.

5. Procédé selon la revendication 1 ou 3, pour la préparation de composés où A signifie propylène.

6. Procédé selon la revendication 1 ou 3, pour la préparation de composés où $R_3$ signifie CN, S-alkyle contenant de 1 à 4 atomes de carbone, $NHSO_2NR_6R_7$, OH ou F.

7. Procédé selon la revendication 1, caractérisé en ce qu'on isole le 2-[N-(3-cyanopropyl)-N-propyl-amino]-1,2,3,4-tétrahydro-5-hydroxy-naphthalène sous forme de base libre ou de sels d'addition d'acides.

8. Procédé selon la revendication 7, pour la préparation des composés sous forme d'antipode (−).

9. Procédé selon la revendication 1, caractérisé en ce qu'on isole le 1,2,3,4-tétrahydro-5-hydroxy-2-[N-(3-méthylthiopropyl)-N-propyl-amino]naphthalène sous forme de base libre ou de sels d'addition d'acides.

10. Procédé selon la revendication 9, pour la préparation des composés sous forme d'antipode (−).

11. Procédé selon la revendication 1, pour la préparation du 1,2,3,4-tétrahydro-5-hydroxy-2-[N-(3-fluoropropyl)-N-propylamino]-naphthalène ou du 1,2,3,4-tetrahydro-5-hydroxy-2-[N-3-chloropropyl)-N-propylamino]-naphthalène sous forme de base libre ou de sels d'addition d'acides.